Europäisches Patentamt
European Patent Office
Office européen des brevets

(11) Publication number: 0 158 796
A2

# EUROPEAN PATENT APPLICATION

(21) Application number: 85102113.9

(51) Int. Cl.[4]: C 12 Q 1/28

(22) Date of filing: 26.02.85

(30) Priority: 27.02.84 US 584013

(43) Date of publication of application: 23.10.85 Bulletin 85/43

(84) Designated Contracting States: AT BE CH DE FR GB IT LI LU NL SE

(71) Applicant: Richardson-Vicks, Inc.
Ten Westport Road
Wilton, CT 06897(US)

(72) Inventor: Shah, Nutan B.
19 Reiner Drive
Shelton Connecticut(US)

(72) Inventor: Chaudhari, Panna R.
17 Spinning Wheel Road
Monroe Connecticut(US)

(74) Representative: Vossius Vossius Tauchner Heunemann Rauh
Siebertstrasse 4 P.O. Box 86 07 67
D-8000 München 86(DE)

(54) **Diagnostic method for detecting periodontal disease.**

(57) Method for detecting the presence of periodontal disease by contacting saliva from a patient suspected of having said disease condition with hydrogen peroxide and a buffered aqueous solution of 4-aminoantipyrine and phenol and observing the color produced as an indication of the presence of periodontal disease.

EP 0 158 796 A2

Our Ref.: T 578 EP
Case: V-1280 EP
Richardson-Vicks Inc.
Connecticut 06897, USA

Feb. 26, 1985
0158796

DIAGNOSTIC METHOD FOR
DETECTING PERIODONTAL DISEASE

## Background of the Invention

Periodontal diseases such as, for example, periodontitis, stomatitis, gingivitis and the like, are inflammatory conditions of the mouth characterized by inflammatory oral tissue changes usually due to local irritation. The destructive inflammatory process involves the interaction between bacteria, food debris, oral leukocytes and the epithelial attachment around the tooth and periodontal membrane, the result of which causes bone resorption (the supportive bone around the roots of the teeth) and inflammation of the tissues (gingiva) immediately surrounding human teeth involving the portion upward from the epithelial attachment (between the surface of gingiva and epithelial attachments only). The early detection of such inflammatory conditions is very important to proper dental treatment. Such detection is herein accomplished by taking advantage of the known fact that, at the site of inflammation, the body produces an increased number of leukocytes such as polymorphonuclear (PMN) leukocytes, which contain a peroxidase enzyme which is a good marker for detecting the presence of the inflammatory periodontal disease condition. When the inflammatory condition is present, the PMN leukocyte count will increase and, concommitantly, so will the peroxidase enzyme activity.

## Detailed Description of Invention

The instant invention provides a simple saliva test for detecting the presence of inflammation due to periodontal disease. It takes advantage of the known chemical ability of peroxidase to catalyze the oxidation of a colorless hydrogen donor (i.e., a chromogen) to a colored product which can be determined colorimetrically. The subject peroxidatic test method is rapid and reliable with a strong positive correlation between the presence of peroxidase in the saliva sample and the existence of such disease.

The determination of peroxidase activity is achieved with a reagent mixture of hydrogen peroxide and 4-aminoantipyrine as the chromogen in a buffered aqueous solution also containing phenol as a coupling agent. The 4-aminoantipyrine is oxidized by the hydrogen peroxide in the presence of peroxidase from the saliva sample and the intensity of the resultant color formation (pink to red) is proportional to the enzyme concentration and, accordingly, to the severity of the periodontal inflammation. The involved chemical reaction may be illustrated as follows:

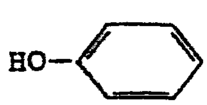 + 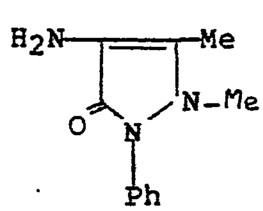 + $H_2O_2$

phenol (coupling agent) | 4-aminoantipyrine (chromogen) | hydrogen peroxide (substrate)

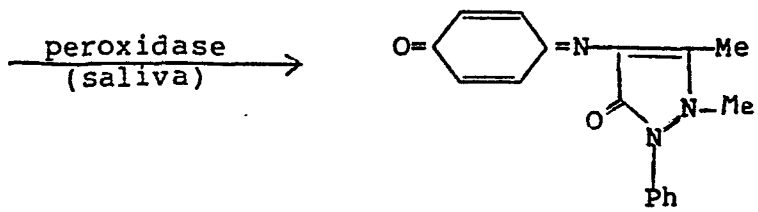


Quinoneimine color complex (chromophore).
Absorption wave length 500 nm ± 15 nm.

The reaction is carried out by mixing (outside the human) measured amounts of the chromogen and substrate solutions with a measured amount of saliva. The reaction is initiated upon contact of the hydrogen peroxide substrate with the saliva so that the order of mixing may first proceed by mixing the chromogen and substrate solutions to which mixture the saliva is then added, or, alternatively, by mixing the chromogen solution and saliva together and then adding the substrate solution.

The end point of the assay will obviously depend on the particular assay conditions employed. For example, increasing the concentration of chromogen and/or substrate, up to an optimum, will enhance the color formation, that is, the time required for observing the color produced by the reaction will be shortened. Thus, one can advantageously adjust the concentrations of chromogen and substrate within the given parameters such that the end point of the assay will be observable within a convenient period of time. Any such adjustment will be readily apparent and easily accomplished by the practitioner of the assay with some familiarization therewith.

Although minimal concentrations of chromogen and substrate may require longer assay times, for example, in excess of 1 hour, it has been generally

found that use of the hereinafter indicated preferred concentrations to the maximum range concentrations will provide suitable assays in about 2 to about 60 minutes and, preferably, in about 5 to about 30 minutes, at room temperature (20-26°C), at which time the color intensity (pink to red) in the reaction mixture is observed. Slightly elevated temperatures up to about 45°C (i.e., below the inactivation temperature of the peroxidase enzyme) may also be employed to enhance the rate of reaction.

The lack of color formation indicates a normal condition, that is, an absence of PMN leukocytes in the saliva which, in turn, indicates the absence of periodontal disease. The formation of color, however, correlates to the presence of periodontal disease and the color intensity correlates to the severity of the disease. A convenient scoring system, from 1 to 4, is as follows:

1 = "normal" - no color developed
2 = "mild" - pink
3 = "moderate" - pinkish red
4 = "severe" - red

The chromogen solution consists of the 4-aminoantipyrine chromogen, the phenol coupling agent and appropriate buffers, preferably conventional alkali metal (sodium or potassium) phosphate buffers, to obtain a desired pH of about 4-9, preferably 6.8 ±0.05, for the reaction conditions.

Chromogen Solution

| Components | % w/v Range | % w/v (pH - 6.8) Preferred |
|---|---|---|
| 4-Aminoantipyrine | 0.001-0.15 g | 0.068 g |
| Phenol | 0.015-1.5 g | 0.55 g |
| Potassium phosphate (monobasic) | 0.15-12.0 g | 5.98 g |
| Potassium phosphate (dibasic) | 0.15-13.0 g | 6.27 g |
| Water, q.s. | 100 ml | 100 ml |

The hydroperoxide substrate solution is preferably a diluted aqueous solution of hydrogen peroxide.

Substrate Solution

| Components | % w/v Range | % w/v Preferred |
|---|---|---|
| Hydrogen peroxide (30%) | 0.001-10 ml | 1.0 ml |
| Water, q.s. | 100 ml | 100 ml |

In general, the method of this invention will utilize about 2-15 unit volumes of the chromogen solution and about 0.1-2 unit volumes of the substrate solution per unit volume of saliva. Optimum efficient amounts may vary, however, depending upon the concentrations of chromogen and/or substrate and the particular assay technique employed in the test. Such techniques, as more fully detailed hereinafter, include test tubes, filter pad sections, procelain wells, spectrophotometers and the like. With the aforementioned preferred chromogen and substrate solutions, we have found, for example, that about 2 unit volumes each of the chromogen solution and the substrate solution per unit volume of saliva is preferred when using test tubes or porcelain wells;

that about 2 unit volumes of the chromogen solution and 1 unit volume of the substrate solution per unit volume of saliva is preferred when using filter pad sections (e.g. discs); and that about 15 unit volumes of the chromogen solution and 0.1 unit volume of the substrate solution per unit volume of saliva is preferred when using spectrophotometric instruments.

To obtain consistently reliable color formation, the pH and peroxide concentration must be controlled. As buffers, there can be used, for example, phosphate (preferred), phthalate, citrate, Tris, borate, succinate and the like buffers, the pH value and capacity thereof being selected in such a manner that the reaction mixture has a pH value of from about 4.0 to about 9.0, and preferably about 6.8. When mixing the chromogen solution and the peroxide substrate solution, it is advantageous to use from about $1.1 \times 10^{-4}$ mole to about 189 moles of 4-aminoantipyrine and from about $1.5 \times 10^{-3}$ mole to about $3.5 \times 10^{3}$ moles of phenol in the chromogen solution for each mole of hydrogen peroxide in the substrate solution. Preferably, the equivalent ratio for each of these reactants is from about 0.031 to about 0.075 mole of 4-aminoantipyrine and from about 0.55 mole to about 1.3 moles of phenol in the chromogen solution for each mole of hydrogen peroxide in the substrate solution.

The method of the invention may be employed in the form of a convenient testing kit providing a stoppered test tube for the chromogen solution, an air-tight container such as a pliable sealed vial or another stopper test tube for the peroxide solution and a calibrated dropper for the saliva sample. The

testing kit may contain, for example, a sufficient amount of the buffered chromogen solution and the peroxide solution for a single test. In conducting the test, the peroxide solution is simply added to the chromogen solution in the test tube followed by a measured sample of saliva,that is, an amount sufficient for the test, previously collected in the dropper to the calibrated measurement. The test tube may then be re-stoppered, shaken and then allowed to stand for the indicated time interval. Means can be provided in the kit for positioning the test tube such that, after the color rection is completed, the resultant color formation may be immediately compared to a color indicator chart also provided with the kit and fromwhich the absence or presence and the severity of the inflammatory periodontal disease can be determined.

Alternate methods include the use of porcelain wells which proivde excellent receptacles for performing the assay with small volumes of reactants and a white background for observing the assay color result; as does the use of a suitable absorbent material, for example, commercially available filter support pads cut into convenient shapes for unit testing, e.g. discs, which maintain all the rectants thereon without leakage and from which the assay color result is readily discernible; and the use of conventional spectrophotometric instruments to automatically "color read" the test assay solutions.

The following examples are given for the purpose of illustrating the invention.

Example 1

Three independent clinical trials were conducted by three research dentists in order to compare and correlate the clinical results thus obtained with the in-vitro results obtained from the subject assay. Each patient was clinically examined to determine the absence or presence of inflammatory periodontal disease and the severity thereof. The result of each clinical diagnosis was scored from 1 (normal) to 4 (severe). A total of 127 male and female subjects participated in the three trials, divided into 65 subjects for Trial I, 39 subjects for Trial II and 23 subjects for Trial III.

In Trial I, each participant was asked to refrain from any oral hygiene or food intake before collecting approximately 5 mls of saliva after awakening in the morning. In Trials II and III, the saliva samples were collected from dental patients at a dental clinic for dental treatment with no prior instructions given regarding any abstinence from oral hygiene or food intake. Each patient was asked to spit once into a spital collecting tube. Each of the saliva samples were analyzed to detect the presence of the peroxidase enzyme according to the following two methods. No significant difference was found between the color scores obtained from both test methods.

A. Disc Method. Absorbent discs measuring 1/4 inch (6.4 mm) in diameter were prepared from filter support pads commercially available from the Millipore Corporation, Bedford, Mass., and identified as

AP 10-037 00, said pads being affixed to the bottom of a Petri dish at spaced intervals by means of an adhesive backing. The test reaction was carried out at room temperature in the following manner:

a. 20 microliters of the previously described Preferred Chromogen Solution was applied to each disc;

b. 10 microliters of the previously described Preferred Substrate Solution was applied to each chromogen treated disc;

c. 10 microliters of the saliva test sample was applied to a thus-treated disc, thereby initiating the test reaction; and

d. 10 minutes after the saliva application in Trial I and 30 minutes after the saliva application in Trials II and III, the presence or absence and intensity of the developed color (pink to red) due to the presence of peroxidase enzyme in the saliva was scored on the aforementioned scale of 1 to 4.

B. <u>Test Tube Method</u>. The test reaction was carried out at room temperature in the following manner:

a. 50 microliters of the Preferred Chromogen Solution was placed in a test tube;

b. 30 microliters of the saliva test sample was added;

c. 60 microliters of the Preferred Substrate Solution was added to initiate the reaction; and

d. (same as step d in the Disc Method).

The results of the clinical diagnosis in the three clinical trials and the results of the in-vitro saliva assay for each of the 127 subjects are shown in Tables I, II and III.

Table 1: Trial I (65 Subjects)

| Patient Number | Clinical Score | In-Vitro Score | Patient Number | Clinical Score | In-Vitro Score | Patient Number | Clinical Score | In-Vitro Score |
|---|---|---|---|---|---|---|---|---|
| 1 | 1 | 1 | 23 | 1-2 | 3 | 45 | 1-2 | 1 |
| 2 | 2 | 2 | 24 | 2 | 4 | 46 | 2 | 2 |
| 3 | 2 | 3 | 25 | 2 | 2 | 47 | 1 | 1 |
| 4 | 2 | 2 | 26 | 1 | 1 | 48 | 3 | 4 |
| 5 | 2 | 2 | 27 | 2 | 2 | 49 | 1-2 | 2 |
| 6 | 2 | 2 | 28 | 1 | 4 | 50 | 1-2 | 1 |
| 7 | 2 | 2 | 29 | 2 | 2 | 51 | 1 | 1 |
| 8 | 4 | 3 | 30 | 1-2 | 3 | 52 | 1-2 | 1 |
| 9 | 3 | 3 | 31 | 1 | 1 | 53 | 1 | 2 |
| 10 | 2 | 3 | 32 | 1 | 2 | 54 | 1 | 2 |
| 11 | 3 | 2 | 33 | 2 | 2 | 55 | 1 | 2 |
| 12 | 1-2 | 1 | 34 | 3 | 4 | 56 | 1-2 | 1 |
| 13 | 2 | 2 | 35 | 3 | 3 | 57 | 2-3 | 2 |
| 14 | 3 | 4 | 36 | 2 | 2 | 58 | 2 | 1 |
| 15 | 1 | 2 | 37 | 1-2 | 1 | 59 | 1-2 | 2 |
| 16 | 2 | 1 | 38 | 2 | 4 | 60 | 2 | 2 |
| 17 | 3 | 2 | 39 | 2 | 4 | 61 | 3 | 4 |
| 18 | 3 | 3 | 40 | 2 | 3 | 62 | 2 | 2 |
| 19 | 1 | 4 | 41 | 3 | 2 | 63 | 1-2 | 2 |
| 20 | 2 | 2 | 42 | 1-2 | 2 | 64 | 1-2 | 2 |
| 21 | 3 | 3 | 43 | 2 | 2 | 65 | 1 | 2 |
| 22 | 4 | 4 | 44 | 1 | 1 | | | |

Table 2: Trial II (39 Subjects)

| Patient Number | Clinical Score | In Vitro Score |
|---|---|---|
| 1 | 3 | 4 |
| 2 | 3 | 1 |
| 3 | 2 | 2 |
| 4 | 3 | 2 |
| 5 | 4 | 2 |
| 6 | 3 | 2 |
| 7 | 2-3 | 3 |
| 8 | 2 | 2 |
| 9 | 3-4 | 2 |
| 10 | 4 | 4 |
| 11 | 3-4 | 1 |
| 12 | 3 | 2 |
| 13 | 3 | 2 |
| 14 | 1 | 1 |
| 15 | 1 | 2 |
| 16 | 1 | 2 |
| 17 | 2 | 3 |
| 18 | 3 | 4 |
| 19 | 4 | 2 |
| 20 | 3 | 4 |
| 21 | 2-3 | 4 |
| 22 | 2 | 4 |
| 23 | 3-4 | 3 |
| 24 | 1 | 2 |
| 25 | 2 | 2 |
| 26 | 1-2 | 2 |
| 27 | 2 | 2 |
| 28 | 4 | 4 |
| 29 | 2 | 2 |
| 30 | 2 | 4 |
| 31 | 1 | 2 |
| 32 | 3-4 | 2 |
| 33 | 2 | 2 |
| 34 | 1 | 1 |
| 35 | 2-3 | 2 |
| 36 | 1 | 2 |
| 37 | 1 | 2 |
| 38 | 1 | 1 |
| 39 | 2 | 3 |

Table 3: Trial III (23 Subjects)

| Patient Number | Clinical Score | In Vitro Score |
|---|---|---|
| 1 | 3 | 1 |
| 2 | 2 | 3 |
| 3 | 2 | 1 |
| 4 | 3 | 2 |
| 5 | 3 | 2 |
| 6 | 4 | 3 |
| 7 | 2-3 | 2 |
| 8 | 3 | 2 |
| 9 | 2 | 2 |
| 10 | 3 | 4 |
| 11 | 2 | 3 |
| 12 | 2-3 | 3 |
| 13 | 3-4 | 4 |
| 14 | 1-2 | 2 |
| 15 | 1-2 | 2 |
| 16 | 1-2 | 2 |
| 17 | 1 | 2 |
| 18 | 1 | 2 |
| 19 | 1 | 2 |
| 20 | 1 | 3 |
| 21 | 1-2 | 3 |
| 22 | 1 | 4 |
| 23 | 1-2 | 3 |

The following Table IV summarizes the foregoing results:

Table 4: Summary

| Clinical Trial | Presence or Absence of Periodontal Inflammation From Clinical & In-Vitro Test | False Positive: Presence of Periodontal Inflammation From In-Vitro Test* | False Negative: Presence of Periodontal Inflammation From Clinical Exam** |
|---|---|---|---|
| I (N=65) | 73.9% | 12.3% | 13.8% |
| II (N=39) | 79.5% | 15.5% | 5.1% |
| III (N=23) | 69.6% | 21.7% | 8.7% |

* The in-vitro test showed the presence of the enzyme peroxidase confirming underlying low level periodontal inflammatory condition; however, no clinical signs of the inflammation were clearly visible upon examination. A "False Positive" occurs when the clinical examination score is 1 and the in-vitro test score is higher than 1.

** The clinical examination recorded inflammation of the gum; however, the in-vitro test did not confirm the presence of the enzyme peroxidase. A "False Negative" occurs when the in-vitro test score is 1 and the clinical examination score is higher than 1.

0158796

## CLAIMS

1. A method for detecting the presence of inflammatory periodontal disease in a human suspected of being afflicted with same which comprises admixing a saliva sample from such human with an aqueous solution of 4-aminoantipyrine and phenol buffered to a pH about 4-9 and with hydrogen peroxide solution and observing the color produced as an indicator of such presence.

2. The method of Claim 1 wherein said pH is about 6.8.

3. A method for detecting the presence of inflammatory periodontal disease in a human suspected of being afflicted with same which comprises admixing a unit volume of saliva sample from such human with about 2-15 unit volumes of a chromogen solution consisting essentially of:

| | % w/v |
|---|---|
| 4-aminoantipyrine | 0.001-0.15 g |
| phenol | 0.015-1.5 g |
| $KH_2PO_4$ (monobasic) | 0.15-12.0 g |
| $K_2H\ PO_4$ (dibasic) | 0.15-13.0 g |
| water, q.s. | 100 ml |

and with about 0.1-2 unit volumes of a substrate solution consisting essentially of:

| | % w/v |
|---|---|
| hydrogen peroxide (30%) | 0.001-10 ml |
| water, q.s. | 100 ml |

and observing the color produced as an indicator of such presence.

4. The method of Claim 3 wherein said chromogen solution consists essentially of:

| | % w/v |
|---|---|
| 4-aminoantipyrine | 0.068 g |
| phenol | 0.55 g |
| $KH_2PO_4$ (monobasic) | 5.98 g |
| $K_2H\ PO_4$ (dibasic) | 6.27 g |
| water, q.s. | 100 ml |

and said substrate solution consists essentially of:

| | % w/v |
|---|---|
| hydrogen peroxide (30%) | 1.0 ml |
| water, q.s. | 100 ml |